# EUROPÄISCHE PATENTSCHRIFT

(11) **EP 3 758 783 B1**
(45) Veröffentlichungstag und Bekanntmachung des Hinweises auf die Patenterteilung: **25.09.2024**
(21) Anmeldenummer: 19708081.5
(22) Anmeldetag: 26.02.2019
(51) Int. Cl.: A61M 25/09, A61M 25/01, A61B 5/055

(54) **FÜHRUNGSDRAHT FÜR MEDIZINISCHE MR-ANWENDUNGEN**
GUIDE WIRE FOR MEDICAL MAGNETIC RESONANCE APPLICATIONS
FIL-GUIDE POUR APPLICATIONS MÉDICALES RM

(30) Priorität: 01.03.2018 DE 102018203102
(43) Veröffentlichungstag der Anmeldung: 06.01.2021
(73) Patentinhaber: EPflex Feinwerktechnik GmbH, 72581 Dettingen/Erms (DE)
(72) Erfinder: UIHLEIN, Bernhard, 72581 Dettingen (DE)
(74) Vertreter: Patentanwälte Ruff, Wilhelm, Beier, Dauster & Partner mbB
(86) Internationale Anmeldenummer: PCT/EP2019/054742
(87) Internationale Veröffentlichungsnummer: WO 2019/166439

(56) Entgegenhaltungen:
- US-A1- 2003 208 142
- US-A1- 2004 059 258
- US-A1- 2010 181 109
- US-A1- 2013 237 963

## Beschreibung

Die Erfindung bezieht sich auf einen Führungsdraht, der zur Verwendung in medizinischen Magnetresonanz(MR-)Anwendungen eingerichtet ist.

Führungsdrähte werden in unterschiedlichen Ausführungen in der invasiven Medizintechnik verwendet, insbesondere zum Einführen in menschliche oder tierische Körperkanäle, um mit dem eingeschobenen Führungsdraht ein anschließendes Einbringen eines Katheterrohres oder dergleichen zu führen. Für Anwendungen in der Magnetresonanztomographie (MRT) bzw. Kernmagnetresonanz(NMR-)Anwendungen oder kurz MR-Anwendungen werden an den Führungsdraht besondere Anforderungen gestellt. Hierfür eignen sich die klassischen Führungsdrähte in der Regel nicht, die einen sich durchgehend von einem proximalen Endbereich zu einem distalen Endbereich des Führungsdrahtes erstreckenden Draht aus einem elektrisch leitenden Metallmaterial, wie einem Edelstahlmaterial oder einer Nickel-Titan(NiTi)-Legierung, beinhalten. Unter anderem kann ein solcher durchgehender Metalldraht zu störenden Artefakten in der MR-Bildgebung und zu unerwünschten Aufheizeffekten durch induktive Erhitzung im Magnetfeld führen. Als Abhilfe sind bereits verschiedentlich Führungsdrähte vorgeschlagen worden, die zur Verwendung in medizinischen MR-Anwendungen eingerichtet sind, siehe beispielsweise die Offenlegungsschriften DE 100 29 738 A1, US 2005/0064223 A1 und DE 10 2011 081 445 A1 sowie die Patentschriften EP 0 864 102 B1 und DE 10 2005 022 688 B4.

Die Offenlegungsschrift DE 10 2007 016 674 A1 offenbart einen Führungsdraht, der axial aus aufeinanderfolgenden Abschnitten gebildet ist, deren Material abwechselnd aus elektrischen Leitern, wie Edelmetalle und NiTi-Legierungen, und Isolatoren, wie Polyurethan, Polyethylen, Polymere und Glasfasern, besteht, wobei die aufeinanderfolgenden Abschnitte miteinander verklebt, verschweißt oder verschraubt sind. Im Inneren des Führungsdrahtes kann ein mit einem Gel oder einer Kontrastflüssigkeit gefüllter Hohlkanal vorgesehen sein, zusätzlich können ein oder mehrere, als Arbeitskanäle fungierende Hohlkanäle gebildet sein.

Die Offenlegungsschrift WO 2007/000148 A2 offenbart einen MR-tauglichen Führungsdraht, der aus einem oder mehreren stabförmigen Körpern und einem nicht-ferromagnetischen Matrixwerkstoff besteht, der den oder die stabförmigen Körper umschließt und/oder miteinander verklebt. Die stabförmigen Körper bestehen aus einem oder mehreren nicht-metallischen Filamenten und einem nicht-ferromagnetischen Werkstoff, der das oder die Filamente umschließt und/oder miteinander verklebt und mit MR-Markerpartikeln dotiert ist. Die Filamente bestehen aus Kunststoff und/oder Glasfaser, der Matrixwerkstoff aus Epoxidharz.

Die Offenlegungsschrift WO 2009/141165 A2 offenbart einen ähnlichen Führungsdraht, der aus einem zentralen stabförmigen Körper und sechs mit Abstand um diesen herum angeordneten stabförmigen Körpern mit gegenüber dem zentralen stabförmigen Körper geringerem Durchmesser aufgebaut ist, wobei die stabförmigen Körper in eine Hüllmatrix eingebettet sind. Die Hüllmatrix besteht aus einem thermoplastischen Elastomer. Die stabförmigen Körper sind aus einem Matrixwerkstoff gebildet, der nicht-metallische Filamente enthält, wobei als Matrixwerkstoff eine Keramik oder ein Kunststoff verwendet wird. Zusätzlich sind die stabförmigen Körper an ihrer Oberfläche mit MR-Markerpartikeln dotiert.

Die Offenlegungsschrift US 2010/0181109 A1 offenbart ein in langgestreckten medizinischen Instrumenten, wie Kathetern und Führungsdrähten, verwendbares Übertragungskabel mit einer Übertragungsleitung und einer Überbrückungseinheit, die jeweils elektrisch leitende und zwischen diesen angeordnete, elektrisch nichtleitende Abschnitte aufweisen, wobei die Überbrückungseinheit relativ zur Übertragungsleitung zwischen einer Stellung, in der ihre elektrisch leitenden Abschnitte die elektrisch nichtleitenden Abschnitte der Übertragungsleitung elektrisch leitend überbrücken, und einer Stellung bewegbar ist, in der die Überbrückungseinheit die elektrisch nichtleitenden Abschnitte der Übertragungsleitung nicht elektrisch leitend überbrückt. Die Übertragungsleitung und die Überbrückungseinheit können z.B. von einer zentrischen Kabeleinheit und einer diese koaxial umgebenden Kabeleinheit gebildet sein.

Der Erfindung liegt als technisches Problem die Bereitstellung eines Führungsdrahtes der eingangs genannten Art zugrunde, der gegenüber dem oben genannten Stand der Technik Vorteile hinsichtlich seines Verhaltens unter MR-Bedingungen, seines Biegeverhaltens bzw. seiner Flexibilität und/oder hinsichtlich eines vergleichsweise geringen Herstellungsaufwands bietet.

Die Erfindung löst dieses Problem durch die Bereitstellung eines Führungsdrahtes mit den Merkmalen des Anspruchs 1.

Der erfindungsgemäße Führungsdraht weist gemäß einem ersten Aspekt der Erfindung einen Multilumendraht aus einem elektrisch nichtleitenden Kunststoffmaterial auf, der sich durchgehend von einem proximalen Endbereich zu einem distalen Endbereich des Führungsdrahtes erstreckt und mindestens zwei getrennte, sich axial erstreckende Hohlkanäle beinhaltet. In mindestens einem der Hohlkanäle ist eine axial abwechselnde Folge von stabförmigen, elastischen Versteifungsstücken aus einem elektrisch leitenden, nicht-magnetischen Material mit gegenüber dem Kunststoffmaterial des Multilumendrahtes höherer Biegesteifigkeit und elektrisch nichtleitenden Abstandsstücken angeordnet.

Gemäß einem weiteren Aspekt der Erfindung, der zusätzlich oder alternativ zum obigen, erstgenannten Aspekt beim erfindungsgemäßen Führungsdraht realisiert sein kann, weist der Führungsdraht mindestens zwei koaxial ineinanderliegend angeordnete, sich durchgehend von einem proximalen Endbereich zu einem distalen Endbereich des Führungsdrahtes erstreckende Drähte bzw. Einzeldrähte auf, vorliegend als Koaxialdrähte bezeichnet. Mindestens zwei der Koaxialdrähte besitzen eine Hohlrohrform und sind aus einer axial abwechselnden Folge von stabförmigen, elastischen Versteifungsstücken aus einem elektrisch leitenden, nicht-magnetischen Material und elektrisch nichtleitenden Abstandsstücken aufgebaut. In entsprechenden Ausführungen sind alle Koaxialdrähte derart aufgebaut, in alternativen Ausführungen sind ein oder mehrere übrige Koaxialdrähte von dem besagten Multilumendraht oder von einem anderen durchgehenden Draht gebildet, der nicht durchgehend elektrisch leitend ist und z.B. aus einem elektrisch nichtleitenden Vollmaterial aus Kunststoff bestehen kann. So kann z.B. ein innerster, zentrischer Koaxialdraht als ein solcher Multilumendraht oder als Kerndraht aus einem elektrisch isolierenden Vollmaterial oder aus abwechselnd aneinandergereihten Versteifungs- und Abstandsstücken aus jeweiligem Vollmaterial gebildet sein, und/oder mindestens ein umgebender Koaxialdraht kann seinerseits als ein solcher Multilumendraht in einer Hohlrohrausführung, bei der sich der oder die Hohlkanäle im Inneren des im Querschnitt ringförmigen Hohlrohrmaterials befinden, oder als Hohlrohrdraht aus einem elektrisch isolierenden Hohlrohrmaterial oder aus abwechselnd aneinandergereihten Versteifungs- und Abstandsstücken aus jeweiligen Hohlrohrteilen bzw. Ringteilen gebildet sein. Der Koaxialdrahtaufbau beinhaltet folglich je nach Ausführung keinen, einen oder mehrere Multilumendrähte der vorliegend betrachteten Art.

Es versteht sich, dass die Erstreckung des Multilumendrahtes und seiner Hohlkanäle bzw. der Koaxialdrähte vom proximalen Endbereich zum distalen Endbereich des Führungsdrahtes den allergrößten Teil der Gesamterstreckung des Führungsdrahtes ausmacht, ohne Ausführungen auszuschließen, bei denen sich an das distale Ende des Multilumendrahtes bzw. der Koaxialdrähte ein kurzer, andersartiger distaler Abschlussabschnitt des Führungsdrahtes anschließt und/oder an das proximale Ende ein kurzer, proximaler Abschlussbereich des Führungsdrahtes anschließt, wie für Führungsdrahtgestaltungen an sich bekannt. Der distale Abschlussbereich kann z.B. eine biegeweichere, distale Endspitze und/oder eine sogenannte J-Spitze des Führungsdrahtes beinhalten. Der proximale Abschlussbereich kann z.B. Kopplungsmittel zum Ankoppeln des Führungsdrahtes an einen Bediengriff oder dergleichen beinhalten. Vorzugsweise umfasst die Erstreckung des Multilumendrahtes und seiner Hohlkanäle bzw. der Koaxialdrähte vom proximalen zum distalen Endbereich des Führungsdrahtes denjenigen Hauptlängenteil des Führungsdrahtes, der im Gebrauch innerhalb eines Körpergewebekanals zu liegen kommt und dort bei MR-Anwendungen den MR-Bedingungen ausgesetzt ist, ausgenommen des besagten distalen Abschlussbereichs.

Der erfindungsgemäße Führungsdraht bietet Vorteile hinsichtlich seines Verhaltens unter MR-Bedingungen, seines Biegeverhaltens bzw. seiner Flexibilität und/oder eines geringen Herstellungsaufwandes. Da der Multilumendraht und die Abstandsstücke in dem oder den betreffenden Hohlkanälen bzw. als Teile des oder der Koaxialdrähte elektrisch nichtleitend sind, lassen sich beim erfindungsgemäßen Führungsdraht lange elektrisch leitende Abschnitte vermeiden, die zu unerwünschten Aufheizeffekten führen könnten. Durch die Verwendung der stabförmigen, elastischen Versteifungsstücke aus elektrisch leitendem, nicht-magnetischem Material lässt sich das Verhalten unter MR-Bedingungen und das Biegeverhalten bzw. die Flexibilität des Führungsdrahtes optimieren. Die Herstellung des Führungsdrahtes mit dem Multilumendraht und den Versteifungsstücken und den Abstandsstücken in dessen Hohlkanal bzw. mit dem Koaxialdrahtaufbau mit mehreren koaxial ineinanderliegenden Koaxialdrähten, von denen wenigstens einer aus den Versteifungsstücken und den Abstandsstücken aufgebaut ist, erfordert in der Regel nur einen vergleichsweise geringen Aufwand.

In einer Weiterbildung der Erfindung besitzt jedes Versteifungsstück eine größere Länge als jedes Abstandsstück. Dies ist für viele Anwendungen bevorzugt. In diesem Fall kann das Biegeverhalten, d.h. das elastische Biegevermögen bzw. die elastische Biegesteifigkeit, des Führungsdrahtes im Wesentlichen durch die Versteifungsstücke bestimmt werden, und die demgegenüber kürzeren Abstandsstücke dienen vorzugsweise primär der elektrischen Unterbrechung bzw. Isolierung aufeinanderfolgender Versteifungsstücke.

In einer Weiterbildung der Erfindung besitzt jedes Versteifungsstück eine Länge zwischen 1cm und 15cm. Mit dieser im Vergleich zu typischen Gesamtlängen von Führungsdrähten relativ geringen Länge lässt sich jegliche Gefahr von Überhitzungseffekten durch induktive Erwärmung der Versteifungsstücke unter MR-Bedingungen zuverlässig vermeiden. Andererseits ist bezüglich des Herstellungsaufwandes eine nicht zu geringe Länge der Versteifungsstücke in der Regel von Vorteil.

In einer Weiterbildung der Erfindung liegt die Länge jedes Abstandsstücks im Bereich zwischen 0,1mm und 5cm. Diese Dimensionierung der Abstandsstücke ist für viele Anwendungsfälle von Vorteil. Oftmals wird eine geringe Länge der Abstandsstücke von z.B. höchstens ca. 1mm oder höchstens ca. 0,5cm oder höchstens ca. 1cm angestrebt.

In vorteilhaften Ausführungen ist die Länge des jeweiligen Versteifungsstücks um mindestens den Faktor fünf, in entsprechenden Realisierungen um mindestens den Faktor zehn größer als die Länge des jeweiligen Abstandsstücks.

In einer Weiterbildung der Erfindung sind die Versteifungsstücke und die Abstandsstücke lose aufeinanderfolgend angeordnet, d.h. lose in den jeweiligen Hohlkanal des Multilumendrahtes eingefügt bzw. lose zur Bildung des betreffenden Koaxialdrahtes aneinandergefügt. Diese Realisierung ist sowohl hinsichtlich Herstellungsaufwand als auch hinsichtlich Funktionszuverlässigkeit sehr vorteilhaft. Durch das lose Anordnen der Versteifungsstücke und der Abstandsstücke entfällt jeglicher Aufwand zur Herstellung entsprechender Verbindungen zwischen den Versteifungsstücken einerseits und den Abstandsstücken andererseits und/oder zwischen dem Multilumendrahtmaterial einerseits und den Versteifungsstücken und/oder den Abstandsstücken andererseits. Dementsprechend besteht im Gebrauch auch keinerlei Gefahr, dass entsprechende Verbindungen brechen oder sich lösen. Zudem können der Multilumendraht, die Versteifungsstücke und die Abstandsstücke jeweils separat vorgefertigt werden, und die Versteifungsstücke und die Abstandsstücke können dann abwechselnd in den jeweiligen Hohlkanal eingeschoben oder zur Bildung des betreffenden Koaxialdrahtes aneinandergereiht bzw. auf einen bestehenden, radial inneren Führungsdrahtteil aufgefädelt werden.

In einer Weiterbildung der Erfindung ist mindestens in einem der Hohlkanäle des Multilumendrahtes ein durchgehender Zugstab aus einem elektrisch nichtleitenden Kunststoffmaterial angeordnet. In einer Weiterbildung der Erfindung ist mindestens einer der Koaxialdrähte von einem durchgehenden Zugstab aus einem elektrisch nichtleitenden Kunststoffmaterial gebildet. In diesen Ausführungen des Führungsdrahtes kann der aus diesem Grund vorliegend so bezeichnete Zugstab die erforderliche Zugfestigkeit des Führungsdrahtes ganz oder jedenfalls zum überwiegenden Teil bereitstellen. Dazu besteht er aus einem geeignet zugfesten bzw. hochfesten Material, wie für derartige Anwendungen in Führungsdrähten an sich bekannt. In entsprechenden Ausführungen besitzt der Zugstab eine geringe Biegesteifigkeit im Vergleich zu den Versteifungsstücken, so dass die Biegesteifigkeit bzw. das Biegevermögen des Führungsdrahtes primär von den Versteifungsstücken bestimmt ist. Alternativ kann der Zugstab so ausgeführt sein, dass er zu einem nicht unerheblichen Teil zusammen mit den Versteifungsstücken zur gewünschten Biegesteifigkeit, d.h. Biegevermögen bzw. Flexibilität, des Führungsdrahtes beiträgt. Da der Zugstab elektrisch nichtleitend ist, verursacht er trotz seiner durchgehenden Erstreckung vom proximalen zum distalen Endbereich des Führungsdrahtes keine störenden bzw. unerwünschten Effekte unter MR-Bedingungen. Der Zugstab kann gleichzeitig mit dem Multilumendraht gefertigt oder separat von diesem gefertigt und nachträglich in den betreffenden Hohlkanal eingeschoben werden. Er kann den Multilumendraht von Zugkraftbelastungen entlasten, d.h. der Multilumendraht braucht in diesem Fall nicht auf die für den Führungsdraht geforderte Zugfestigkeit hin ausgelegt werden.

In einer Ausgestaltung der Erfindung gehört zu den Hohlkanälen im Multilumendraht ein mittiger Hohlkanal, in welchem der durchgehende Zugstab aus elektrisch nichtleitendem Kunststoffmaterial angeordnet ist. In einer alternativen Ausgestaltung der Erfindung gehört zu den Koaxialdrähten ein mittiger Koaxialdraht, der von dem durchgehenden Zugstab aus einem elektrisch nichtleitenden Kunststoffmaterial gebildet ist. Die zentrische Anordnung des die Zugfestigkeit des Führungsdrahtes bestimmenden Zugstabes ist für viele Anwendungen vorteilhaft.

In einer Weiterbildung der Erfindung gehört zu den Hohlkanälen im Multilumendraht ein mittiger Hohlkanal, in welchem die abwechselnde Folge der Versteifungsstücke und Abstandsstücke angeordnet ist. In einer alternativen Weiterbildung der Erfindung gehört zu den Koaxialdrähten ein mittiger Koaxialdraht, der durch die abwechselnde Folge der Versteifungsstücke und Abstandsstücke gebildet ist. Diese Ausführungen können für bestimmte Anwendungsfälle Vorteile z.B. hinsichtlich weitgehend richtungsunabhängiger Biegefestigkeit bieten, einschließlich den Fällen, in denen kein zentrischer Zugstab vorgesehen ist.

In einer Weiterbildung der Erfindung ist für mehrere der Hohlkanäle und/oder Koaxialdrähte die abwechselnde Folge der Versteifungsstücke und Abstandsstücke angeordnet, und die Versteifungsstücke und Abstandsstücke sind in einem ersten dieser Hohlkanäle und/oder Koaxialdrähte gegenüber den Versteifungsstücken und Abstandsstücken in einem zweiten dieser Hohlkanäle und/oder Koaxialdrähte axial versetzt angeordnet. Dies kann zur Erzielung einer möglichst gleichmäßigen Biegesteifigkeit, d.h. eines möglichst gleichmäßigen Biegeverhaltens, des Führungsdrahtes längs seiner Axialerstreckung vorteilhaft sein. Zudem kann dadurch bei Bedarf das Verhalten des Führungsdrahtes unter MR-Bedingungen optimiert werden.

In einer Ausgestaltung der Erfindung sind die Abstandsstücke im Führungsdraht, d.h. in den Hohlkanälen bzw. für die Koaxialdrähte, axial überlappungsfrei angeordnet. Dies bedeutet, dass an jedem beliebigen Punkt längs der axialen Erstreckung des Führungsdrahtes im Querschnitt des Führungsdrahtes höchstens ein Abstandsstück vorhanden ist, wenngleich mehrere Hohlkanäle und/oder Koaxialdrähte durch die abwechselnde Folge von Versteifungsstücken und Abstandsstücken befüllt bzw. aufgebaut sind. Umgekehrt bedeutet dies, dass an jedem Punkt längs der axialen Erstreckung des Führungsdrahtes im Querschnitt mindestens eines der Versteifungsstücke vorhanden ist. Diese Eigenschaften sind für viele Anwendungsfälle hinsichtlich Verhalten unter MR-Bedingungen und Biegeverhalten vorteilhaft.

In einer Weiterbildung der Erfindung gehört zu den Hohlkanälen im Multilumendraht eine Mehrzahl von außermittigen Hohlkanälen, in denen die abwechselnde Folge der Versteifungsstücke und Abstandsstücke angeordnet ist. Dies kann insbesondere zur Erzielung eines allseitig möglichst gleichmäßigen Biegeverhaltens des Führungsdrahtes vorteilhaft sein, insbesondere wenn in entsprechenden Ausführungen die außermittigen Hohlkanäle in Umfangsrichtung mit gleichmäßigem Winkelabstand voneinander um eine Längsmittenachse des Führungsdrahtes herum angeordnet sind.

In einer Weiterbildung der Erfindung ist der Multilumendraht oder ein äußerster der Koaxialdrähte von einem Schrumpfschlauch umgeben. Diese Ausführung kann bei Bedarf vorteilhaft dazu genutzt werden, durch die zusammenpressende Wirkung des Schrumpfschlauchs die Versteifungsstücke und die Abstandsstücke gegen unbeabsichtigte Axialbewegung gesichert in ihrer Lage festzuhalten. Dadurch lässt sich zuverlässig vermeiden, dass die Versteifungsstücke und die Abstandsstücke verrutschen bzw. sich axial wegbewegen, auch wenn sie lose in den Führungsdraht eingefügt sind. Der Schrumpfschlauch kann zudem mit seiner Außenseite eine gewünschte Oberfläche des Führungsdrahtes bereitstellen, z.B. eine hydrophile oder antithrombogene Oberfläche.

In einer Weiterbildung der Erfindung ist der Multilumendraht oder ein äußerster der Koaxialdrähte außenseitig mit einer hydrophilen Beschichtung oder einer antithrombogenen Beschichtung versehen. Dies kann für entsprechende Führungsdrahtanwendungen von Vorteil sein.

In einer Weiterbildung der Erfindung sind die Versteifungsstücke aus einer Nickel-Titan-Legierung oder einem Edelstahlmaterial gebildet. Die so gebildeten Versteifungsstücke zeigen das von diesen Materialien an sich bekannte, vorteilhafte Biegeverhalten.

In einer Weiterbildung der Erfindung ist der Multilumendraht aus einem thermoplastischen Kunststoffmaterial gebildet. Dazu kann er mittels an sich bekannter, vorteilhaft einfacher Fertigungsverfahren hergestellt werden. Das thermoplastische Kunststoffmaterial kann z.B. ein Polyurethan- oder Polyamid-Material sein.

In einer Weiterbildung der Erfindung sind die Abstandsstücke aus einem thermoplastischen Kunststoffmaterial, z.B. das gleiche oder ein anderes Material als dasjenige für den Multilumendraht, oder einem duroplastischen Kunststoffmaterial oder einem Keramikmaterial gebildet. Beispielsweise ist auch ein Polytetrafluorethylen(PTFE-)Material verwendbar.

In einer Weiterbildung der Erfindung sind in den Führungsdraht MR- Markerpartikel eingebracht. Die MR-Markerpartikel können z.B. in einem oder mehreren der Hohlkanäle und/oder an oder in den Versteifungsstücken, den Abstandsstücken und/oder dem durchgehenden Zugstab und/oder im Material des Multilumendrahtes und/oder in einem distalen Spitzenbereich des Führungsdrahtes, der sich an das distale Ende des Multilumendrahtes bzw. Koaxialdrahtaufbaus anschließt, angeordnet sein. Mit relativ geringem Aufwand kann eine durchgehende MR-Sichtbarkeit des Führungsdrahtes z.B. dadurch realisiert sein, dass der durchgehende Zugstab verwendet wird und auf diesem die MR-Markerpartikel angebracht werden, vorzugsweise in gleichmäßigen Abständen. Bei Bedarf kann eine Röntgensichtbarkeit des Führungsdrahtes vorzugsweise in dessen distalem Endbereich durch entsprechendes lokales Anbringen eines röntgensichtbaren Federelementes oder eines anderen röntgensichtbaren Elementes unterstützt werden.

In einer Weiterbildung der Erfindung ist wenigstens eines der Versteifungsstücke an einem oder beiden Enden verrundet und/oder in seiner Biegesteifigkeit verringert, letzteres z.B. durch entsprechendes Verjüngen oder Warmbehandeln. Beide Maßnahmen können in entsprechenden Führungsdrahtanwendungen je für sich und in Kombination einer Schädigung des Führungsdrahtes durch scharfkantige Stirnenden des Versteifungsstücks in einem im Gebrauch gekrümmten Bereich des Führungsdrahtes vorbeugen. Die endseitige Biegesteifigkeitsreduzierung des Versteifungsstücks kann zudem bei Bedarf ein Biegen des Führungsdrahtes erleichtern.

Vorteilhafte Ausführungsformen der Erfindung sind in den Zeichnungen dargestellt. Diese und weitere Ausführungsformen der Erfindung werden nachfolgend näher beschrieben. Hierbei zeigen:
- Fig. 1: eine Perspektivansicht eines hier interessierenden Teils eines Führungsdrahtes mit einem Multilumendrahtaufbau mit einem mittigen und sechs außermittigen Hohlkanälen, alle mit einer Füllung aus abwechselnden Versteifungsstücken und Abstandsstücken,
- Fig. 2: eine Perspektivdarstellung des Führungsdrahtes von Fig. 1 mit transparenter Wiedergabe des Multilumendrahtes zur besseren Sichtbarkeit der Versteifungsstücke und Abstandsstücke,
- Fig. 3: eine Abwicklungsdarstellung der mit den Versteifungsstücken und Abstandsstücken gefüllten Hohlkanäle des Mulitlumendrahtaufbaus von Fig. 1,
- Fig. 4: eine Querschnittansicht längs einer Linie IV-IV von Fig. 1,
- Fig. 5: die Querschnittansicht von Fig. 4 für eine Variante mit zentrischem, durchgehendem Zugstab,
- Fig. 6: die Abwicklungsansicht von Fig. 3 für die Variante von Fig. 5,
- Fig. 7: die Querschnittansicht von Fig. 4 für eine Variante mit vier außermittigen Hohlkanälen,
- Fig. 8: die Querschnittansicht von Fig. 4 für eine Variante mit drei außermittigen Hohlkanälen,
- Fig. 9: die Querschnittansicht von Fig. 4 für eine Variante mit zwei außermittigen Hohlkanälen,
- Fig. 10: die Querschnittansicht von Fig. 4 für eine Variante mit mittigem Hohlkanal mit durchgehendem Zugstab und vier außermittigen Hohlkanälen größeren Durchmessers,
- Fig. 11: die Querschnittansicht von Fig. 4 für eine Variante mit mittigem Hohlkanal mit durchgehendem Zugstab und acht außermittigen Hohlkanälen kleineren Durchmessers,
- Fig. 12: die Querschnittansicht von Fig. 4 für eine Variante mit mittigem Hohlkanal mit durchgehendem Zugstab und zwölf außermittigen Hohlkanälen kleineren Durchmessers,
- Fig. 13: die Querschnittansicht von Fig. 4 für eine Variante mit mittigem Hohlkanal mit durchgehendem Zugstab und achtzehn auf zwei Radien angeordneten, außermittigen Hohlkanälen,
- Fig. 14: eine Längsschnittansicht eines hier interessierenden Teils eines Führungsdrahtes mit einem Koaxialdrahtaufbau,
- Fig. 15: eine Querschnittansicht längs einer Linie XV-XV von Fig. 14,
- Fig. 16: eine Längsschnittansicht eines proximalen Endabschnitts eines Führungsdrahtes mit einem Multilumendrahtaufbau und proximaler Abschlusskuppe aus einem Klebematerial,
- Fig. 17: die Längsschnittansicht von Fig. 16 für eine Variante mit proximaler Abschlusskuppe aus aufgeschmolzenem Multilumendrahtmaterial,
- Fig. 18: eine Längsschnittansicht eines distalen Endabschnitts eines Führungsdrahtes mit Multilumendrahtaufbau mit konisch verjüngtem distalem Multilumendrahtende,
- Fig. 19: die Längsschnittansicht von Fig. 18 für eine Variante mit distal gestuft endenden Versteifungsstücken,
- Fig. 20: eine Seitenansicht eines im erfindungsgemäßen Führungsdraht verwendbaren Versteifungsstücks mit halbkugelförmig gerundeten Enden,
- Fig. 21: eine Seitenansicht eines im erfindungsgemäßen Führungsdraht verwendbaren Versteifungsstücks mit konisch verjüngten und abgerundeten Enden,
- Fig. 22: eine Seitenansicht eines im erfindungsgemäßen Führungsdraht verwendbaren Versteifungsstücks mit konisch verjüngten und endkugelbestückten Enden und
- Fig. 23: eine Seitenansicht eines im erfindungsgemäßen Führungsdraht verwendbaren Versteifungsstücks mit konisch verjüngten und endpaddelbestückten Enden.

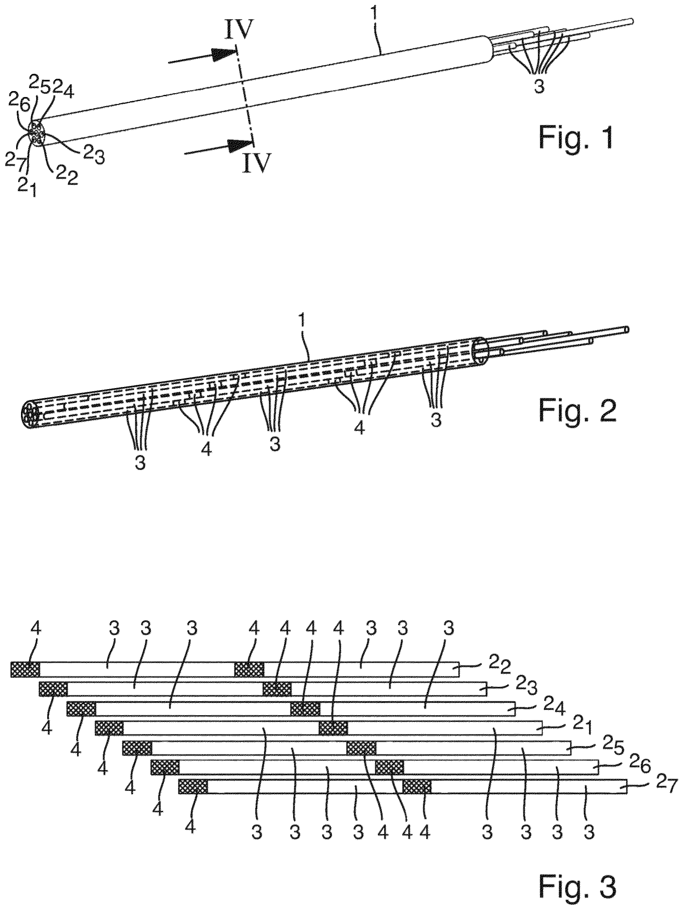

Der in den Fig. 1 bis 4 in einem hier interessierenden, repräsentativen Abschnitt veranschaulichte Führungsdraht ist von einem Multilumendrahtaufbau mit einem Multilumendraht 1 aus einem elektrisch nichtleitenden Kunststoffmaterial. Der Multilumendraht 1 erstreckt sich durchgehend von einem proximalen Endbereich zu einem distalen Endbereich des Führungsdrahtes, wobei er hier nur mit einem repräsentativen Teilabschnitt seiner Länge gezeigt ist. Er weist mindestens zwei getrennte, sich axial erstreckende Hohlkanäle 2₁, 2₂, ..., 2ₙ auf, mit n als beliebiger natürlicher Zahl größer als eins.

Im gezeigten Ausführungsbeispiel der Fig. 1 bis 4 beinhaltet der Multilumendraht 1 sieben Hohlkanäle 2₁ bis 2₇, speziell einen mittigen Hohlkanal 2₁ und sechs außermittige Hohlkanäle 2₂ bis 2₇, die vorzugsweise auf einem gemeinsamen Radius, alternativ in einer anderen regelmäßigen oder unregelmäßigen Konfiguration, um den mittigen Hohlkanal 2₁ herum angeordnet sind. Im gezeigten Beispiel erstrecken sich alle Hohlkanäle 2₁ bis 2₇ axial geradlinig im Grundkörpermaterial des Multilumendrahtes 1 aus Kunststoff, in alternativen Ausführungen erstrecken sich einer oder manche der Hohlkanäle oder alle Hohlkanäle schraubenförmig gewunden. Der Multilumendraht 1 aus dem Kunststoffmaterial, das vorzugsweise eine relativ geringe Biegesteifigkeit besitzt, kann durch irgendeines der hierfür an sich bekannten Fertigungsverfahren hergestellt werden, z.B. mittels eines Extrusionsverfahrens.

Mindestens in einem der Hohlkanäle 2₁ bis 2₇ des Multilumendrahtes 1 ist eine axial abwechselnde Folge von stabförmigen, elastischen Versteifungsstücken 3 und Abstandsstücken 4 angeordnet. Die Versteifungsstücke 3 bestehen aus einem elektrisch leitenden, nicht-magnetischen Material mit gegenüber dem Kunststoffmaterial des Multilumendrahtes höherer Biegesteifigkeit. Die Abstandsstücke 4 bestehen aus einem elektrisch nichtleitenden Material. Vorzugsweise liegen die Versteifungsstücke 3 und die Abstandsstücke 4 ohne größeres Radialspiel in den Hohlkanälen 2₁ bis 2₇, d.h. in diesbezüglichen Ausführungen ist der Außendurchmesser der Versteifungsstücke 3 und der Abstandsstücke 4 annähernd so groß wie der Innendurchmesser der Hohlkanäle 2₁ bis 2₇.

Insgesamt resultiert daher aus diesem Multilumendrahtaufbau, dass der Führungsdraht nicht durchgehend von seinem proximalen zu seinem distalen Endbereich elektrisch leitend ist, sondern lediglich die Versteifungsstücke 3 elektrisch leitende Bereiche des Führungsdrahtes bilden, die voneinander durch die Abstandsstücke 4 elektrisch isoliert sind. In den Fig. 1 bis 4 ist der Führungsdraht mit einer Teillänge dargestellt, die in jedem Hohlkanal 2₁ bis 2₇ jeweils zwei Versteifungsstücke 3 und zwei Abstandsstücke 4 umfasst. Auf seiner nicht gezeigten übrigen Länge vom proximalen zum distalen Endbereich besitzt der Führungsdraht einen Aufbau, der einer periodischen Fortsetzung der gezeigten Teillänge entspricht. Elektrisch leitend sind jeweils nur die elektrisch voneinander isolierten, einzelnen Versteifungsstücke 3. Jedes Versteifungsstück 3 kann z.B. aus einem Vollstab- bzw. Vollrohrmaterial bestehen, alternativ aus einem Hohlstab- bzw. Hohlrohrmaterial. Ebenso können die Abstandsstücke 4 z.B. aus einem Vollmaterial oder alternativ einem Hohlmaterial bestehen.

In entsprechenden Ausführungen des Führungsdrahtes besitzt jedes Versteifungsstück 3 eine größere Länge als jedes Abstandsstück 4. In alternativen Ausführungen ist mindestens ein Abstandsstück 4 länger als mindestens ein Versteifungsstück 3. Die Versteifungsstücke 3 besitzen in entsprechenden Ausführungen untereinander eine gleiche Länge, in alternativen Ausführungen sind mindestens zwei Versteifungsstücke 3 unterschiedlich lang. Ebenso besitzen die Abstandsstücke 4 in entsprechenden Ausführungen untereinander eine gleiche Länge, in alternativen Ausführungen besitzen mindestens zwei Abstandsstücke 4 unterschiedliche Längen. Bei den oben genannten Dimensionierungsangaben für die Längen der Versteifungsstücke und der Abstandsstücke bleiben etwaige Teilstücke am distalen und am proximalen Ende des Multilumendrahtaufbaus außer Betracht, die sich ergeben können, wenn die Versteifungsstücke und Abstandsstücke in verschieden Hohlkanälen versetzt angeordnet sind und am distalen und proximalen Ende die abwechselnde Folge von Versteifungsstücken und Abstandsstücken in allen Hohlkanälen auf gleicher axialer Höhe abschließen soll.

In vorteilhaften Ausführungen liegt die Länge jedes Versteifungsstücks 3 im Bereich zwischen 1cm und 15cm oder spezieller zwischen 5cm und 10cm. In entsprechenden Ausführungen liegt die Länge jedes Abstandsstücks 4 im Bereich zwischen 1mm und 5cm oder spezieller zwischen 1mm und 1cm. In entsprechenden Ausführungen ist die Länge des jeweiligen Versteifungsstücks 3 um mindestens den Faktor fünf oder spezieller um mindestens den Faktor zehn größer als die Länge des jeweiligen Abstandsstücks 4.

In vorteilhaften Ausführungsformen sind die Versteifungsstücke 3 und die Abstandsstücke 4 im jeweiligen Hohlkanal 2₁ bis 2₇ lose aufeinanderfolgend angeordnet, d.h. lose in selbigen eingefügt. In vielen Anwendungsfällen brauchen die Versteifungsstücke 3 und die Abstandsstücke 4 nicht aneinander und nicht mit dem Grundkörpermaterial des Multilumendrahtes 1 an den betreffenden Hohlkanalwandungen durch Verbindungsmittel, wie Klebe- oder Schweißverbindungen, fixiert werden. Dies spart zugehörigen Herstellungsaufwand ein. Es genügt, bei Bedarf die Hohlkanäle 2₁ bis 2₇ an ihrem distalen und ihrem proximalen Ende zu verschließen, so dass die Versteifungsstücke 3 und die Abstandsstücke 4 dort nicht herausgelangen können. Zur Fertigung des Führungsdrahtes kann der Multilumendraht 1 als entsprechender Grundkörper aus Kunststoffmaterial vorgefertigt werden, und die separat vorgefertigten Versteifungsstücke 3 und Abstandsstücke 4 können dann abwechselnd in den jeweiligen Hohlkanal 2₁ bis 2₇ eingeschoben werden. In einer alternativen Ausführung ist jeweils ein Abstandsstück 4 an einem Versteifungsstück 3 fixiert, z.B. durch eine Verschweißung oder durch Ankleben eines in diesem Fall als Abstandsstück 4 fungierenden Klebepunktes aus einem elektrisch isolierenden Klebematerial an ein Stirnende des stabförmigen Versteifungsstücks 3. Diese Kombinationsstücke aus je einem Versteifungsstück 3 und Abstandsstück 4 können vorgefertigt und dann in die Hohlkanäle 2₁ bis 2₇ eingeschoben werden. In einer Variante kann jeweils ein Abstandsstück 4 an beiden Stirnenden eines Versteifungsstücks 3 fixiert werden, und diese Kombinationsstücke können dann abwechselnd mit Versteifungsstücken 3 ohne vormontiertes Abstandsstück 4 in die Hohlkanäle 2₁ bis 2₇ eingeschoben werden.

In entsprechenden Realisierungen sind die Versteifungsstücke 3 aus einer NiTi-Legierung, wie z.B. Nitinol, oder einem Edelstahlmaterial gebildet. Die Abstandsstücke 4 können z.B. aus einem thermoplastischen Kunststoffmaterial oder einem duroplastischen Kunststoffmaterial oder einem Keramikmaterial gebildet sein, speziell z.B. aus PTFE. Die Versteifungsstücke 3 dienen in der Regel primär zur Bereitstellung einer gewünschten elastischen Biegesteifigkeit bzw. Biegefähigkeit des Führungsdrahtes. In diesen Fällen besitzt der Multilumendraht 1 eine gegenüber den Versteifungsstücken 3 deutlich geringere Biegesteifigkeit, und die Abstandsstücke 4 sind ebenfalls biegeweicher als die Versteifungsstücke 3 und/oder tragen wegen einer deutlich geringeren Länge nicht signifikant zur Biegesteifigkeit des Führungsdrahtes bei. Der Multilumendraht 1 ist vorzugsweise aus einem thermoplastischen Kunststoffmaterial gebildet. Er ist optional außenseitig mit einer hydrophilen Beschichtung oder einer antithrombogenen Beschichtung versehen.

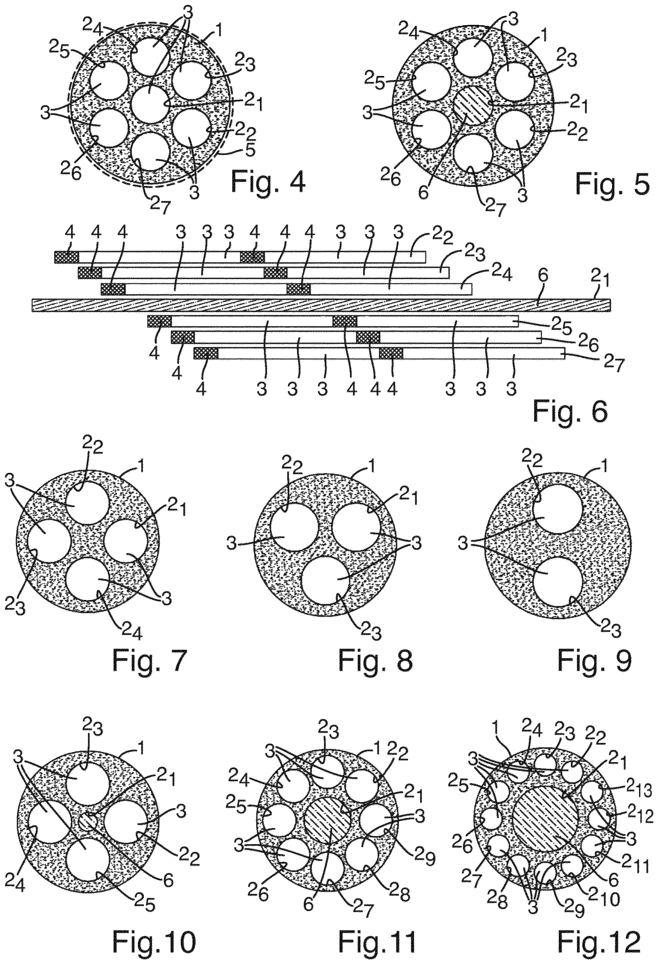

Optional ist der Multilumendraht 1 von einem in Fig. 4 gestrichelt angedeuteten Schrumpfschlauch 5 umgeben. Der Schrumpfschlauch 5 kann bei Bedarf dazu genutzt werden, das Grundkörpermaterial des Multilumendrahtes 1 radial nach innen zusammenzupressen. Dies kann bei Bedarf das axiale Festhalten der Versteifungsstücke 3 und der Abstandsstücke 4 in den Hohlkanälen 2₁ bis 2₇ unterstützen.

In entsprechenden Ausführungsformen sind in den Führungsdraht und spezieller in den Multilumendrahtaufbau MR-Markerpartikel eingebracht. Diese können sich z.B. in einem oder mehreren der Hohlkanäle 2₁ bis 2₇ und/oder an oder in den Versteifungsstücken 3 und/oder an oder in den Abstandsstücken 4 und/oder im Material des Multilumendrahtes 1 befinden.

In vorteilhaften Ausführungsformen des Erfindungsdrahtes ist, wie im Ausführungsbeispiel der Fig. 1 bis 4, in mehreren der Hohlkanäle 2₁ bis 2₇ die abwechselnde Folge der Versteifungsstücke 3 und Abstandsstücke 4 angeordnet, wobei die Versteifungsstücke 3 und Abstandsstücke 4 in einem ersten dieser Hohlkanäle, z.B. im Hohlkanal 2₁, gegenüber den Versteifungsstücken 3 und Abstandsstücken 4 in einem zweiten dieser Hohlkanäle, z.B. im Hohlkanal 2₂, axial versetzt angeordnet sind, wie insbesondere aus den Fig. 2 und 3 ersichtlich. In der Ausführung der Fig. 1 bis 4 sind die Versteifungsstücke 3 und Abstandsstücke 4 in einem der Hohlkanäle 2₁ bis 2₇ um die Länge des Abstandsstücks 4 gegenüber den Versteifungsstücken 3 und Abstandsstücken 4 in einem anderen der Hohlkanäle 2₁ bis 2₇ versetzt, d.h. die Abstandsstücke 4 folgen in der Abwicklungsdarstellung von Fig. 3 von einem zu einem nächsten Hohlkanal 2₁ bis 2₇ lückenlos aufeinander, mit Ausnahme einer etwaigen Lücke eines in der Abwicklungsdarstellung von Fig. 3 letzten Hohlkanals, z.B. dem Hohlkanal 2₇, zu einem ersten Hohlkanal, z.B. dem Hohlkanal 2₂, bei dem dann die nächste Sequenz von Abstandsstücken 4 und Versteifungsstücken 3 beginnt.

Wie speziell aus Fig. 3 ersichtlich, sind im Ausführungsbeispiel der Fig. 1 bis 4 die Abstandsstücke 4 im Führungsdraht axial überlappungsfrei angeordnet, d.h. an jedem beliebigen Punkt des Führungsdrahtes entlang seiner axialen Länge ist im Querschnitt des Führungsdrahtes höchstens ein Abstandsstück 4 vorhanden. Da in diesem Beispiel alle Hohlkanäle 2₁ bis 2₇ lückenlos mit der abwechselnden Folge von Versteifungsstücken 3 und Abstandsstücken 4 befüllt sind, bedeutet dies umgekehrt, dass an jedem beliebigen Punkt längs der Axialerstreckung des Führungsdrahtes im Querschnitt immer mindestens sechs Versteifungsstücke 3 vorhanden sind. Ein derartiger Aufbau trägt dazu bei, dass der Führungsdraht längs seiner Axialerstreckung eine sehr gleichmäßige Steifigkeit bzw. ein sehr gleichmäßiges Biegemoment aufweist.

In Ausführungen, in denen wie im Beispiel der Fig. 1 bis 4 alle Hohlkanäle 2₁ bis 2₇ mit lose eingebrachten Versteifungsstücken 3 und Abstandsstücken 4 belegt sind, werden Zugkraftbelastungen vom Grundkörper-Kunststoffmaterial des Multilumendrahtes 1 übernommen, d.h. dieser Multilumendraht-Grundkörper aus Kunststoff wird in diesem Fall zur Bereitstellung der geforderten Zugfestigkeit des Führungsdrahtes ausgelegt.

In den Fig. 5 bis 13 sind verschiedene Ausführungsvarianten des Führungsdrahtes der Fig. 1 bis 4 mit Multilumendrahtaufbau veranschaulicht. Speziell zeigen die Fig. 5 und 6 ein Ausführungsbeispiel, das demjenigen der Fig. 1 bis 4 mit der einzigen Modifikation entspricht, dass im mittigen Hohlkanals 2₁ anstelle der Folge von abwechselnden Versteifungsstücken 3 und Abstandsstücken 4 ein durchgehender Zugstab 6 aus einem elektrisch nichtleitenden Kunststoffmaterial hoher Festigkeit angeordnet ist.

Der durchgehende Zugstab 6 kann Zugkräfte aufnehmen, so dass der Multilumendraht 1 mit seinem Grundkörpermaterial im Hinblick auf Zugkraftanforderungen entlastet werden kann, wenn gewünscht. Der durchgehende Zugstab 6 ist in der Regel relativ biegeweich ausgeführt, so dass er die durch die Versteifungsstücke 3 definierte Biegesteifigkeit des gesamten Führungsdrahtes nicht signifikant beeinflusst.

Der durchgehende Zugstab 6 kann z.B. aus einem hochfesten Kunststoffmaterial bestehen, wie es zur Verwendung in Führungsdrähten z.B. als zentrischer Kerndraht an sich bekannt ist. Optional sind MR-Markerpartikel am durchgehenden Zugstab 6 angeordnet, z.B. an seiner Oberfläche. Die MR-Markerpartikel können bevorzugt auf der gesamten oder einem überwiegenden Teil der Länge des Zugstabes 6 durchgängig oder in vorzugsweise regelmäßigen Abständen angeordnet sein.

In nicht gezeigten, alternativen Ausführungen ist der durchgehende Zugstab 6 in einem der außermittigen Hohlkanäle 2₂ bis 2₇ angeordnet, oder es sind mehrere solche durchgehende Zugstäbe 6 in mehreren, beliebig ausgewählten Hohlkanälen angeordnet, wobei in letztem Fall die abwechselnde Folge von Versteifungsstücken 3 und Abstandsstücken 4 nur noch in einem oder mehreren übrigen Hohlkanälen angeordnet ist.

Eine in Fig. 7 veranschaulichte Ausführungsform unterscheidet sich von derjenigen der Fig. 1 bis 4 darin, dass der Multilumendraht 1 nur vier außermittige Hohlkanäle 2₁ bis 2₄ aufweist, die vorzugsweise in einem gleichen 90°-Winkelabstand um eine Längsmittenachse des Führungsdrahtes herum im Grundkörper-Kunststoffmaterial des Multilumendrahtes 1 gebildet sind. In diesem gezeigten Beispiel sind alle vier Hohlkanäle 2₁ bis 2₄ mit der abwechselnden Folge von Versteifungsstücken 3 und Abstandsstücken 4 versehen. In alternativen Ausführungen können stattdessen in einem, zwei oder drei der Hohlkanäle 2₁ bis 2₄ ein bzw. mehrere durchgehende Zugstäbe 6 der oben zum Ausführungsbeispiel der Fig. 5 und 6 erläuterten Art vorgesehen sein.

Eine in Fig. 8 gezeigte Ausführungsvariante entspricht derjenigen von Fig. 7 mit der Ausnahme, dass nur drei außermittige Hohlkanäle 2₁, 2₂, 2₃ im Multilumendraht 1 vorgesehen sind. Vorzugsweise sind die drei Hohlkanäle 2₁, 2₂, 2₃, wie gezeigt, in einem gleichmäßigen 120°-Winkelabstand um eine Längsmittenachse des Führungsdrahtes herum angeordnet. In der gezeigten Ausführung sind wiederum alle Hohlkanäle 2₁, 2₂, 2₃ mit der abwechselnden Folge von Versteifungsstücken 3 und Abstandsstücken 4 gefüllt. In alternativen Ausführungen ist stattdessen in einem oder zwei dieser drei Hohlkanäle 2₁, 2₂, 2₃ der erwähnte durchgehende Zugstab 6 angeordnet.

In einer in Fig. 9 dargestellten Ausführungsvariante besitzt der Multilumendraht 1 nur zwei außermittige Hohlkanäle 2₁, 2₂. In der gezeigten Ausführung ist in beiden Hohlkanälen 2₁, 2₂ die jeweilige abwechselnde Folge der Versteifungsstücke 3 und Abstandsstücke 4 angeordnet. In einer alternativen Ausführung ist stattdessen in einem der Hohlkanäle 2₁, 2₂ der durchgehende Zugstab 6 angeordnet.

In den Ausführungsbeispielen der Fig. 1 bis 9 haben alle Hohlkanäle 2₁ bis 2₇ des Multilumendrahtes 1 einen kreisrunden Querschnitt mit gleichem Durchmesser. In alternativen Ausführungen haben die Hohlkanäle eine andere Querschnittsform, z.B. einen ovalen, elliptischen oder mehreckigen Querschnitt, und/oder sie besitzen voneinander unterschiedliche Querschnittsformen und/oder Querschnittsflächen. Derartige Ausführungsvarianten sind in den Fig. 10 bis 12 veranschaulicht.

Bei der Ausführungsvariante von Fig. 10 besitzt der Multilumendraht 1 einen mittigen Hohlkanal 2₁ mit kleinerem Querschnitt und vier außermittige Hohlkanäle 2₂ bis 2₅ mit untereinander gleichem und gegenüber dem ersten Hohlkanal 2₁ größerem Querschnitt bzw. Durchmesser. Wie im Beispiel von Fig. 5 ist im mittigen Hohlkanal 2₁ der durchgehende Zugstab 6 angeordnet, der in diesem Fall einen kleineren Querschnitt besitzt, während in den außermittigen Hohlkanälen 2₂ bis 2₅ die abwechselnde Folge der Versteifungsstücke 3 und Abstandsstücke 4 angeordnet ist. Die Versteifungsstücke 3 und die Abstandsstücke 4 besitzen in diesem Fall einen größeren Querschnitt als der durchgehende Zugstab 6.

Bei einer in Fig. 11 gezeigten Ausführungsvariante weist der Multilumendraht 1 einen mittigen Hohlkanal 2₁ mit größerem Querschnitt und acht außermittige Hohlkanäle 2₂ bis 2₉ mit gegenüber dem mittigen Hohlkanal 2₁ geringerem Querschnitt auf. Wiederum ist in den mittigen Hohlkanal 2₁ ein durchgehender Zugstab 6 eingebracht, hier mit entsprechend größerem Querschnitt, während in den außermittigen Hohlkanälen 2₂ bis 2₉, die wiederum mit vorzugsweise äquidistantem Winkelabstand um den mittigen Hohlkanal 2₁ herum angeordnet sind, die abwechselnde Folge von Versteifungsstücken 3 und Abstandsstücken 4 angeordnet ist.

Bei der in Fig. 12 veranschaulichten Ausführungsvariante beinhaltet der Multilumendraht 1 einen mittigen Hohlkanal 2₁ mit relativ großem Querschnitt und zwölf außermittige Hohlkanäle 2₂ bis 2₁₃ mit demgegenüber deutlich kleinerem Querschnitt, die wiederum in vorzugsweise äquidistantem Winkelabstand um den mittigen Hohlkanal 2₁ herum angeordnet sind. Im mittigen Hohlkanal 2₁ ist wiederum der durchgehende Zugstab 6 angeordnet, hier mit entsprechend großem Querschnitt, und die außermittigen Hohlkanäle 2₂ bis 2₁₃ sind mit der abwechselnden Folge von Versteifungsstücken 3 und Abstandsstücken 4 versehen. In weiteren, nicht gezeigten, alternativen Ausführungsformen ist der durchgehende Zugstab 6 der Ausführungsbeispiele gemäß den Fig. 10 bis 12 durch die abwechselnde Folge von Versteifungsstücken 3 und Abstandsstücken 4 ersetzt, und/oder es sind ein oder mehrere durchgehende Zugstäbe 6 in einem oder mehreren der außermittigen Hohlkanäle angeordnet, wobei jeweils noch in mindestens einem der Hohlkanäle die abwechselnde Folge der Versteifungsstücke 3 und Abstandsstücke 4 angeordnet ist.

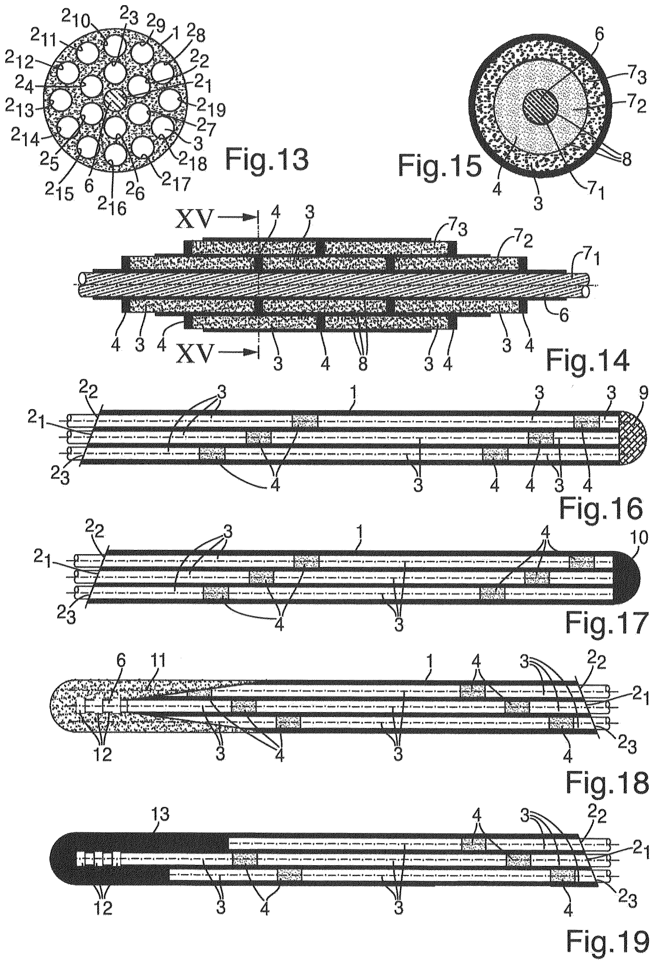

Bei einer in Fig. 13 veranschaulichten Ausführungsform besitzt der Multilumendraht 1 einen mittigen Hohlkanal 2₁, eine erste Gruppe außermittiger Hohlkanäle, z.B. wie gezeigt sechs solche Hohlkanäle 2₂ bis 2₇, die auf einem ersten Radius um den mittigen Hohlkanal 2₁ herum angeordnet sind, und eine zweite Gruppe außermittiger Hohlkanäle, die auf einem zweiten Radius um die erste Gruppe von Hohlkanälen herum angeordnet ist, z.B. wie gezeigt zwölf solche Hohlkanälen 2₈ bis 2₁₉

Im gezeigten Beispiel von Fig. 13 ist im mittigen Hohlkanal 2₁ der erwähnte durchgehende Zugstab 6 angeordnet, während die übrigen Hohlkanäle 2₂ bis 2₁₉ mit der abwechselnden Folge von Versteifungsstücken 3 und Abstandsstücken 4 belegt sind. In alternativen Ausführungen ist der durchgehende Zugstab 6 im mittigen Hohlkanal 2₁ durch die Folge von Versteifungsstücken 3 und Abstandsstücken 4 ersetzt und/oder in einem oder mehreren der außermittigen Hohlkanäle 2₂ bis 2₁₉ ist der durchgehende Zugstab 6 anstelle der abwechselnden Folge von Versteifungsstücken und Abstandsstücken 4 angeordnet. Im gezeigten Beispiel besitzen alle Hohlkanäle 2₁ bis 2₁₉ einen gleichen, kreisrunden Querschnitt, in alternativen Ausführungen unterscheiden sich mindestens zwei der Hohlkanäle 2₁ bis 2₁₉ in ihrer Querschnittsform und/oder in ihrer Querschnittsfläche. In der Regel ist eine größere Anzahl von Hohlkanälen und folglich eine größere Anzahl von darin aufgenommenen Versteifungsstücken 3 und Abstandsstücken 4 bzw. durchgehenden Zugstäben 6 hinsichtlich Erzielung eines gleichmäßigen Biegeverhaltens des Führungsdrahtes von Vorteil.

Die Fig. 14 und 15 veranschaulichen einen Führungsdraht mit Koaxialdrahtaufbau. Dieser beinhaltet mindestens zwei koaxial ineinanderliegend angeordnete, sich durchgehend von einem proximalen Endbereich zu einem distalen Endbereich des Führungsdrahtes erstreckende Koaxialdrähte 7₁, ..., 7ₙ, mit n als einer beliebigen natürlichen Zahl größer als eins. Im gezeigten Beispiel der Fig. 14 und 15 sind dies speziell drei Koaxialdrähte 7₁, 7₂, 7₃, in alternativen Ausführungen nur zwei oder mehr als drei Koaxialdrähte.

Mindestens einer der Koaxialdrähte 7₁ bis 7ₙ ist aus einer axial abwechselnden Folge von stabförmigen, elastischen Versteifungsstücken 3 aus einem elektrisch leitenden, nicht-magnetischen Material und elektrisch nichtleitenden Abstandsstücken 4 aufgebaut. Die Versteifungsstücke 3 entsprechen in Material, Form und Funktion denjenigen der oben erwähnten Führungsdrähte mit Multilumendrahtaufbau, und ebenso entsprechen die Abstandsstücke 4 in Material, Form und Funktion denjenigen der oben erwähnten Führungsdrähte mit Multilumendrahtaufbau.

Im Ausführungsbeispiel der Fig. 14 und 15 bildet ein erster Koaxialdraht 7₁ einen mittigen, als Kerndraht fungierenden Koaxialdraht. Der mittige Koaxialdraht 7₁ ist aus Vollmaterial, alternativ aus einem Hohlrohrmaterial, gebildet. Im gezeigten Beispiel ist der mittige Koaxialdraht 7₁ speziell durch einen sich durchgehend vom proximalen Endbereich zum distalen Endbereich des Führungsdrahtes erstreckenden Zugstab 6 der oben zu den Führungsdrähten mit Multilumendrahtaufbau erläuterten Art gebildet, was eine Ausführung des Zugstabes 6 aus Voll- oder Hohlstabmaterial umfasst.

Im gezeigten Ausführungsbeispiel der Fig. 14 und 15 ist der mittige Koaxialdraht 7₁ von einem zweiten Koaxialdraht 7₂ koaxial umgeben, der seinerseits von einem dritten Koaxialdraht 7₃ koaxial umgeben ist. Insgesamt ergibt sich der besagte Koaxialdrahtaufbau als ein Rohr-in-Rohr-Aufbau, wobei der zweite und der dritte Koaxialdraht 7₂, 7₃ jeweils eine Hohlrohrform besitzen, um den radial nach innen anschließenden Koaxialdraht im Inneren aufzunehmen. Zwischen den aneinandergrenzenden Koaxialdrähten 7₁, 7₂, 7₃ ist vorzugsweise eine elektrisch isolierende Beschichtung oder ein Schrumpfschlauchmaterial 8 vorgesehen. Eine solche Beschichtung bzw. ein solches Schrumpfschlauchmaterial 8 ist optional zusätzlich an der Außenseite des äußersten Koaxialdrahtes 7₃ vorgesehen, wobei es sich in diesem letztgenannten Fall vorzugsweise auch um eine hydrophile oder antithrombogene Beschichtung handeln kann.

Im gezeigten Beispiel der Fig. 14 und 15 sind der mittlere, zweite Koaxialdraht 7₂ und der äußere, dritte Koaxialdraht 7₃ jeweils aus der axial abwechselnden Folge von Versteifungsstücken 3 und Abstandsstücken 4 gebildet. Dazu besitzen die Versteifungsstücke 3 und die Abstandsstücke 4 jeweils eine entsprechende Hohlrohr- bzw. Ringform. Zur Fertigung des Führungsdrahtes können z.B. einzelne hohlrohr- bzw. ringförmige Versteifungsstücke 3 und Abstandsstücke 4 vorgefertigt und auf den mittigen Koaxialdraht 7₁ bzw. den bereits gebildeten, radial inneren Koaxialdrahtaufbau mit dem mittigen, inneren Koaxialdraht 7₁ und dem zweiten, mittleren Koaxialdraht 7₂ aufgefädelt werden.

Für die Dimensionierung und das Anordnen der Versteifungsstücke 3 und der Abstandsstücke 4 sind beim Führungsdraht mit dem Koaxialdrahtaufbau die oben zu den Führungsdrähten mit Multilumendrahtaufbau erläuterten Ausführungsvarianten in entsprechender Weise realisierbar, so dass hierzu auf die obigen Ausführungen verwiesen werden kann. Dies gilt insbesondere hinsichtlich Materialwahl und Längenerstreckung für die Versteifungsstücke 3 einerseits und die Abstandsstücke 4 andererseits. Durch den Koaxialdrahtaufbau lässt sich ein richtungsabhängig sehr gleichbleibendes Biegeverhalten für den Führungsdraht erzielen. Auch beim Koaxialdrahtaufbau sind in entsprechenden Ausführungen die Versteifungsstücke 3 und die Abstandsstücke 4, wenn sie für mehrere Koaxialdrähte vorgesehen sind, bei mindestens einem dieser Koaxialdrähte vorzugsweise axial versetzt bezüglich denjenigen bei mindestens einem der anderen Koaxialdrähte angeordnet. Im gezeigten Beispiel der Fig. 14 und 15 ist eine mittige Versetzung der Versteifungsstücke 3 und Abstandsstücke 4 in ihren Anordnungen für den zweiten und dritten Koaxialdraht 7₂, 7₃ realisiert. Des Weiteren sind in entsprechenden Ausführungen die Abstandsstücke 4 im Führungsdraht axial überlappungsfrei angeordnet, wie dies auch im Beispiel der Fig. 14 und 15 der Fall ist.

In nicht gezeigten Ausführungsvarianten des Koaxialdrahtaufbaus der Fig. 14 und 15 besteht der Führungsdraht nur aus zwei ineinanderliegenden Koaxialdrähten oder aus mehr als drei ineinanderliegenden Koaxialdrähten. In zum Ausführungsbeispiel der Fig. 14 und 15 alternativen Ausführungen ist die abwechselnde Folge der Versteifungsstücke 3 und Abstandsstücke 4 in einem der beiden betreffenden Koaxialdrähte 7₂, 7₃ durch den durchgehenden Zugstab 6 ersetzt, der in diesem Fall mit einer geeigneten Hohlstabform gebildet ist. In einer weiteren alternativen Ausführung ist der mittige Koaxialdraht 7₁ statt durch den Zugstab 6 durch die abwechselnde Folge von Versteifungsstücken 3 und Abstandsstücken 4 gebildet. In diesem Fall sind die Versteifungsstücke 3 und Abstandsstücke 4 vorzugsweise aus Vollmaterial gebildet.

In weiteren Ausführungsformen sind der Multilumendrahtaufbau und der Koaxialdrahtaufbau miteinander kombiniert. In einer diesbezüglichen Realisierung besitzt der Führungsdraht den Koaxialdrahtaufbau nach Art der Fig. 14 und 15 mit zwei oder einer beliebigen größeren Anzahl von Koaxialdrähten 7₁ bis 7ₙ, wobei der mittige Koaxialdraht 7₁ durch den Multilumendraht 1 aus elektrisch nichtleitendem Kunststoffmaterial mit den mindestens zwei axialen Hohlkanälen 2₁ bis 2ₙ gebildet ist, wobei sich in mindestens einem dieser Hohlkanäle 2₁ bis 2ₙ des Multilumendrahtes 1 die abwechselnde Folge von Versteifungsstücken 3 und Abstandsstücken 4 befindet. In alternativen Realisierungen sind ein oder mehrere der umgebenden Koaxialdrähte 7₂ bis 7ₙ durch den Multilumendraht 1 gebildet, der in diesem Fall in einer entsprechenden Hohlrohrausführung bereitgestellt ist und die sich axial erstreckenden Hohlkanäle 2₁ bis 2ₙ in seinem Hohlrohrmaterial aufweist, wobei wiederum mindestens einer dieser Hohlkanäle 2₁ bis 2ₙ die abwechselnde Folge von Versteifungsstücken 3 und Abstandsstücken 4 enthält.

Allgemein kann bei diesen Kombinationsausführungen der Führungsdraht aus einer Mehrzahl von Koaxialdrähten bestehen, von denen mindestens einer vom Multilumendraht 1 mit der abwechselnden Folge von Versteifungsstücken 3 und Abstandsstücken 4 in mindestens einem seiner Hohlkanäle 2₁ bis 2ₙ und optional mit dem durchgehenden Zugstab 6 in etwaigen übrigen Hohlkanälen und die übrigen Koaxialdrähte durch die abwechselnde Folge von Versteifungsstücken 3 und Abstandsstücken 4 oder durch den durchgehenden Zugstab 6 gebildet sind.

Ein in Fig. 16 veranschaulichter Führungsdraht besitzt einen Multilumendrahtaufbau der oben erläuterten Art, wobei der Multilumendraht 1 an seinem proximalen Ende von einer halbkugelförmigen Kuppe 9 aus einem Klebematerial abgeschlossen ist. Die Abschlusskuppe 9 verschließt proximal die Hohlkanäle 2₁ bis 2ₙ des Multilumendrahtes 1 und verhindert dadurch auch ein etwaiges proximales Herausgelangen der in den Hohlkanälen 2₁ bis 2ₙ aufgenommenen Versteifungsstücke 3 und Abstandsstücke 4.

Die Fig. 17 veranschaulicht eine Ausführungsvariante des Führungsdrahtes von Fig. 16. Bei dieser Variante ist ebenfalls eine halbkugelförmige, proximale Abschlusskuppe 10 vorgesehen, die in diesem Fall jedoch aus aufgeschmolzenem Kunststoffmaterial des Multilumendrahtes 1 besteht. Es braucht daher für diese Realisierung kein zusätzliches Klebematerial angebracht werden. Ansonsten entspricht die Abschlusskuppe 10 von Fig. 17 der proximalen Abschlusskuppe 9 von Fig. 16 in Form und Funktion.

Fig. 18 veranschaulicht in einem hier interessierenden distalen Endabschnitt einen Führungsdraht mit einem Multlumendrahtaufbau der oben erwähnten Art, bei dem der Multilumendraht 1 samt der in den Hohlkanälen 2₁ bis 2ₙ eingebrachten Versteifungsstücke 3 und Abstandsstücke 4 an seinem distalen Ende konisch verjüngt ausgebildet ist, z.B. durch einen entsprechenden Abschleifprozess. Dadurch ergibt sich für diesen Führungsdraht eine gewünschte geringere Biegesteifigkeit des distalen Endabschnitts.

In an sich bekannter Weise ist an den konisch verjüngten Endteil des Multilumendrahtes 1 eine distale Endkappe 11 aus einem geeigneten, biegeweichen Füllmaterial angefügt. Dadurch wird ein konstanter Durchmesser des Führungsdrahtes bis zu dessen distalem Abschluss beibehalten. Optional sind am distalen Spitzenende des Multilumendrahtes 1, wie gezeigt, oder alternativ am oder im Material der distalen Endkappe 11 ein oder mehrere MR-Marker 12 angeordnet. In vorteilhaften Ausführungen dieser Art besitzt der Multilumendraht 1 einen mittigen Hohlkanal mit darin eingebrachtem, durchgehendem Zugstab 6. Bevorzugt steht der Zugstab 6 in diesen Fällen distal über den konischen Teil des distalen Spitzenende des Multilumendrahtes 1 hinaus vor und trägt die MR-Marker 12 und/oder trägt zu einem sicheren Halt der Endkappe 11 und/oder zur Sicherstellung einer ausreichenden Zugfestigkeit des Führungsdrahtes auch in diesem distalen Abschlussbereich bei. In optionalen, nicht gezeigten Ausführungen kann im distalen Endabschnitt des Führungsdrahtes ein die Röntgensichtbarkeit verbesserndes Element vorgesehen sein, z.B. eine auf das distale Ende des Multilumendrahtes 1 bzw. des zentrischen Zugstabes aufgeschobene bzw. in das distale Endkappen-Füllmaterial eingebettete Schraubenfeder.

Fig. 19 zeigt eine Variante des Führungsdrahtes von Fig. 18, bei welcher die gegenüber dem proximal anschließenden Führungsdrahtbereich geringere Biegesteifigkeit des distalen Führungsdrahtendabschnitts durch abgestuftes Einbringen der abwechselnden Folge von Versteifungsstücken 3 und Abstandsstücken 4 in die verschiedenen Hohlkanäle 2₁ bis 2ₙ statt durch konisches Verjüngen des Multilumendrahtes 1 realisiert ist. Dies bedeutet, dass die jeweils distal letzten Versteifungsstücke 3 in den betroffenen Hohlkanälen 2₁ bis 2ₙ auf unterschiedlicher axialer Höhe distal enden.

Als distaler Abschluss des Führungsdrahtes fungiert wiederum eine distale Endkappe 13 aus einem geeigneten Füllmaterial, wobei durch dieses Füllmaterial auch eine durch das gestufte Anordnen der distal letzten Versteifungsstücke 3 verbleibende distale Restlänge in den betroffenen Hohlkanälen verschlossen werden kann. Das Füllmaterial für die distale Abschlusskappe 13 kann demjenigen für die distale Abschlusskappe 11 in der Variante von Fig. 18 entsprechen oder von diesem verschieden sein. In einer entsprechenden Realisierung ist das Füllmaterial für die distale Abschlusskappe 13 aus dem Kunststoffmaterial des Multilumendrahtes 1 gebildet. Optional kann der Multilumendraht 1wiederum einen mittigen Hohlkanal mit dem darin eingebrachten Zugdraht 6 aufweisen, der dann analog zum Ausführungsbeispiel von Fig. 18 vorzugsweise das sich am weitesten distal nach vorn erstreckende Drahtelement bildet und für die benötigte Zugfestigkeit des distalen Abschlussbereichs sorgt sowie bei Bedarf als Träger der MR-Marker 12 fungieren kann.

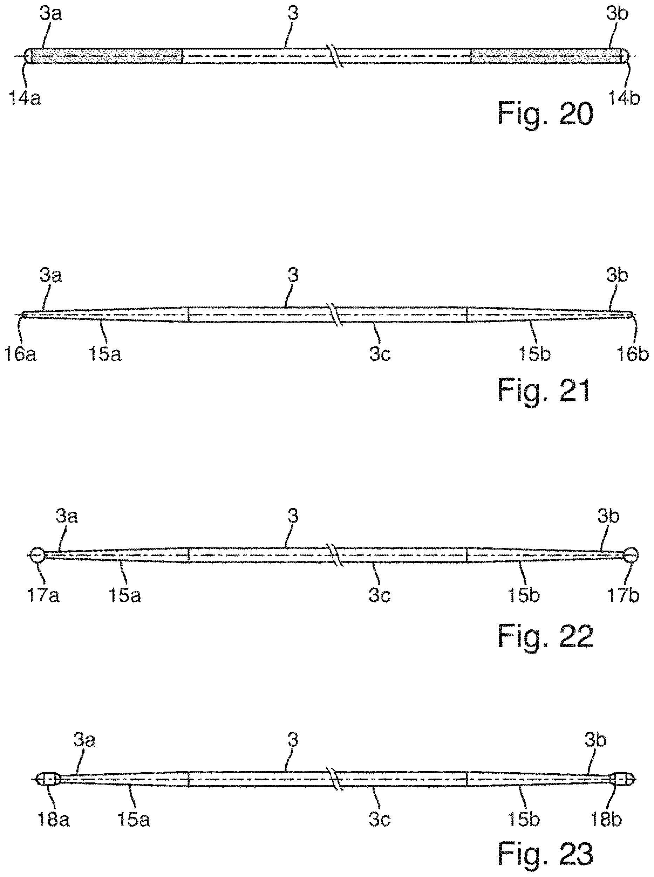

Wie bereits erwähnt, bestehen die Versteifungsstücke 3 aus einem entsprechenden Vollstab- bzw. Vollrohrmaterial oder Hohlstab- bzw. Hohlrohrmaterial. Dazu können sie z.B. durch Ablängen eines entsprechenden Vollstabs bzw. Vollrohres oder Hohlstabs bzw. Hohlrohres gefertigt sein. In entsprechenden Ausführungen kann das jeweilige Versteifungsstück 3 in einer endbehandelten Realisierung verwendet werden, gemäß der es an einem seiner beiden Enden oder an beiden Enden verrundet und/oder in seiner Biegesteifigkeit verringert ist. Dadurch kann bei Bedarf das Biegeverhalten des Führungsdrahtes in einer gewünschten Weise beeinflusst und/oder Schädigungen des Führungsdrahtes insbesondere im Fall stärkerer Krümmungsbelastungen des Führungsdrahtes vorgebeugt werden. Die Fig. 20 bis 23 veranschaulichen hierzu vier exemplarische Beispiele, bei denen das Versteifungsstück 3 jeweils beidseitig endbehandelt ist. In alternativen Ausführungen ist das Versteifungsstück 3 nur an einem seiner beiden Enden auf diese Weise endbehandelt. In entsprechenden Ausführungsformen des Führungsdrahtes ist mindestens eines der in ihm verwendeten Versteifungsstücke 3 endbehandelt, vorzugsweise mehrere seiner Versteifungsstücke 3 einschließlich des Falles, dass alle seine Versteifungsstücke 3 endbehandelt sind.

Im Ausführungsbeispiel der Fig. 20 ist das Versteifungsstück 3 an seinen beiden Enden 3a, 3b jeweils dadurch verrundet, dass es dort stirnseitig mit einer halbkugelförmigen Abschlusskuppe 14a, 14b abschließt. Die Abschlusskuppe 14a, 14b kann fertigungstechnisch einfach z.B. durch Aufschmelzen des Versteifungsstückmaterials an der Stirnseite des jeweiligen Endes 3a, 3b des Versteifungsstücks 3 gebildet werden. Durch die Abschlusskuppen 14a, 14b endet das Versteifungsstück 3 axial nicht mit einer abrupten Stirnrandkante, wodurch sich verhindern lässt, dass eine solche Stirnrandkante ein umgebendes Material verletzt bzw. durchbohrt, wie ein umgebendes Hohlkanalmaterial oder eine Außenhaut des Führungsdrahtes, wenn sich das Versteifungsstück 3 mit seinem betreffenden Ende 3a, 3b in einem Bereich befindet, in welchem der Führungsdraht relativ stark gekrümmt wird.

Fig. 21 veranschaulicht ein Ausführungsbeispiel, bei dem das Versteifungsstück 3 an seinen beiden Enden 3a, 3b konisch verjüngt ist, d.h. es ist jeweils ein konisch verjüngter Endbereich 15a, 15b gebildet, dessen Durchmesser sich von einem konstanten Durchmesser des Versteifungsstücks 3 in einem Mittenbereich 3c zum zugehörigen Stirnende hin verringert, vorzugsweise stufenlos, alternativ in einer oder mehreren Stufen. Optional ist wie im gezeigten Beispiel der konisch verjüngte Endbereich 15a, 15b an seinem Stirnendabschluss verrundet, z.B. wiederum mit einer halbkugelförmigen Abschlusskuppe 16a, 16b. Durch diese graduelle endseitige Verjüngung wird die Biegesteifigkeit des Versteifungsstücks 3 am entsprechenden Ende 3a, 3b verringert. Dadurch kann das Versteifungsstück 3 in diesem Bereich einer Biegung bzw. Krümmung des Führungsdrahtes leichter nachgeben bzw. folgen, wodurch das Versteifungsstück 3 weniger stark entgegen der Krümmungsrichtung radial nach außen gegen angrenzendes Material, wie ein Multilumendrahtmaterial des Führungsdrahtes oder eine Außenhaut des Führungsdrahtes, drückt, was etwaigen Verletzungen bzw. Schädigungen dieses Materials durch das Versteifungsstück 3 insbesondere in gekrümmten Abschnitten des Führungsdrahtes vorbeugt. Zu Letzterem trägt zudem die stirnseitige Verrundung der sich konisch verjüngenden Bereiche 15a, 15b durch die Abschlusskuppen 16a, 16b bei.

Fig. 22 veranschaulicht ein Ausführungsbeispiel, das demjenigen von Fig. 21 in der Bildung der sich konisch verjüngenden Bereiche 15a, 15b entspricht und sich von diesem darin unterscheidet, dass der Stirnendabschluss der sich konisch verjüngenden Bereiche 15a, 15b jeweils durch eine verrundende Abschlusskugel 17a, 17b gebildet ist, deren Durchmesser größer ist als der angrenzende minimale Durchmesser des sich konisch verjüngenden Bereichs 15a, 15b. Diese endseitige Verdickung kann etwaigen Schädigungen des Führungsdrahtes bei stärkerer Krümmung desselben vorbeugen, insbesondere verhindern, dass der sich konisch verjüngende Bereich 15a, 15b mit seinem verjüngten Ende ein umgebendes Material, wie eine Ummantelung oder Außenhaut des Führungsdrahtes, verletzt oder durchbohrt, wenn der Führungsdraht in diesem Bereich gekrümmt wird.

Das in Fig. 23 gezeigte Ausführungsbeispiel entspricht mit den sich konisch verjüngenden Bereichen 15a, 15b des Versteifungsstücks 3 den Beispielen der Fig. 21 und 22, wobei in diesem Fall der jeweilige sich konisch verjüngende Bereich 15a, 15b nicht bis zum Stirnende des Versteifungsstücks 3 ausgebildet ist, sondern mit etwas Abstand davon endet und über einen axial kurzen, sich konisch verbreiternden Bereich in den ursprünglichen Durchmesser des für die Herstellung des Versteifungsstücks 3 benutzten Stabrohlings übergeht, um anschließend analog zum Ausführungsbeispiel von Fig. 20 wieder mit einer halbkugelförmigen Abschlusskuppe abzuschließen, so dass sich insgesamt jeweils ein paddelförmiger Endabschluss 18a, 18b für das Versteifungsstück 3 ergibt. Auch in diesem Fall ist in vorteilhafter Weise eine endseitig reduzierte Biegesteifigkeit des Versteifungsstücks 3 mit einer vor Schädigungen schützenden Endverrundung kombiniert.

Optional kann bei jeder der in den Fig. 20 bis 23 veranschaulichten Ausführungen zusätzlich eine Warmbehandlung eines oder beider Enden 3a, 3b des Versteifungsstücks 3 vorgesehen sein, insbesondere durch Weichglühen bzw. Erhöhen der sogenannten AF-Temperatur superelastischer Legierungen wie NiTi-Legierungen. Diese Warmbehandlung bewirkt ebenfalls eine Verringerung der Biegesteifigkeit des Versteifungsstücks 3 an seinem betreffenden Ende 3a, 3b bzw. Endbereich.

Wie die gezeigten und oben erläuterten Ausführungsbeispiele deutlich machen, stellt die Erfindung in sehr vorteilhafter Weise einen Führungsdraht für medizinische MR-Anwendungen zur Verfügung, der in seinem Verhalten unter MR-Bedingungen optimiert ist und insbesondere unerwünschte Aufheizeffekte unter MR-Bedingungen vermeidet. Der Führungsdraht lässt sich mit vergleichsweise geringem Herstellungsaufwand fertigen und bietet eine hohe Funktionszuverlässigkeit. Durch geeignete Systemauslegung kann er bei Bedarf in seinem Biegeverhalten bzw. in seiner Flexibilität auf den jeweiligen Anwendungsfall optimal abgestimmt werden.

## Patentansprüche

1. Führungsdraht, der zur Verwendung in medizinischen Magnetresonanzanwendungen eingerichtet ist, mit
- einem Multilumendraht (1) aus einem elektrisch nichtleitenden Kunststoffmaterial, der sich durchgehend von einem proximalen Endbereich zu einem distalen Endbereich des Führungsdrahtes erstreckt und mindestens zwei getrennte, sich axial erstreckende Hohlkanäle (2₁,..., 2ₙ) aufweist, wobei mindestens in einem der Hohlkanäle des Multilumendrahtes eine axial abwechselnde Folge von stabförmigen, elastischen Versteifungsstücken (3) aus einem elektrisch leitenden, nicht-magnetischen Material mit gegenüber dem Kunststoffmaterial des Multilumendrahtes höherer Biegesteifigkeit und elektrisch nichtleitenden Abstandsstücken (4) angeordnet ist, und/oder
- koaxial ineinanderliegend angeordneten, sich durchgehend von einem proximalen Endbereich zu einem distalen Endbereich des Führungsdrahtes erstreckenden Koaxialdrähten (7₁,..., 7ₙ), wobei mindestens zwei der Koaxialdrähte (7₂, 7₃) eine Hohlrohrform besitzen und aus einer axial abwechselnden Folge von stabförmigen, elastischen Versteifungsstücken (3) aus einem elektrisch leitenden, nicht-magnetischen Material und elektrisch nichtleitenden Abstandsstücken (4) aufgebaut sind.

2. Führungsdraht nach Anspruch 1, wobei
- jedes Versteifungsstück eine größere Länge als jedes Abstandsstück besitzt und/oder
- die Länge jedes Versteifungsstücks im Bereich zwischen 1cm und 15cm liegt und/oder
- die Länge jedes Abstandsstücks im Bereich zwischen 0,1mm und 5cm liegt.

3. Führungsdraht nach Anspruch 1 oder 2, wobei die Versteifungsstücke und die Abstandsstücke lose aufeinanderfolgend angeordnet sind.

4. Führungsdraht nach einem der Ansprüche 1 bis 3, wobei mindestens in einem der Hohlkanäle ein durchgehender Zugstab (6) aus einem elektrisch nichtleitenden Kunststoffmaterial angeordnet ist.

5. Führungsdraht nach Anspruch 4, wobei zu den Hohlkanälen ein mittiger Hohlkanal gehört, in dem der durchgehende Zugstab aus elektrisch nichtleitendem Kunststoffmaterial angeordnet ist.

6. Führungsdraht nach einem der Ansprüche 1 bis 5, wobei mindestens einer der Koaxialdrähte von einem durchgehenden Zugstab (6) aus einem elektrisch nichtleitenden Kunststoffmaterial gebildet ist.

7. Führungsdraht nach Anspruch 6, wobei zu den Koaxialdrähten ein mittiger Koaxialdraht gehört, der von dem durchgehenden Zugstab aus einem elektrisch nichtleitenden Kunststoffmaterial gebildet ist.

8. Führungsdraht nach einem der Ansprüche 1 bis 7, wobei
- zu den Hohlkanälen ein mittiger Hohlkanal gehört, in dem die abwechselnde Folge der Versteifungsstücke und Abstandsstücke angeordnet ist, oder
- zu den Koaxialdrähten ein mittiger Koaxialdraht gehört, der von der abwechselnden Folge der Versteifungsstücke und Abstandsstücke gebildet ist.

9. Führungsdraht nach einem der Ansprüche 1 bis 8, wobei für mehrere der Hohlkanäle und/oder Koaxialdrähte die abwechselnde Folge der Versteifungsstücke und Abstandsstücke angeordnet ist und die Versteifungsstücke und Abstandsstücke in einem ersten dieser Hohlkanäle und/oder Koaxialdrähte gegenüber den Versteifungsstücken und Abstandsstücken in einem zweiten dieser Hohlkanäle und/oder Koaxialdrähte axial versetzt angeordnet sind.

10. Führungsdraht nach Anspruch 9, wobei die Abstandsstücke im Führungsdraht axial überlappungsfrei angeordnet sind.

11. Führungsdraht nach einem der Ansprüche 1 bis 10, wobei zu den Hohlkanälen eine Mehrzahl von außermittigen Hohlkanälen gehört, in denen die abwechselnde Folge der Versteifungsstücke und Abstandsstücke angeordnet ist.

12. Führungsdraht nach einem der Ansprüche 1 bis 11, wobei der Multilumendraht oder ein äußerster der Koaxialdrähte von einem Schrumpfschlauch (5) umgeben oder außenseitig mit einer hydrophilen Beschichtung oder einer antithrombogenen Beschichtung versehen ist.

13. Führungsdraht nach einem der Ansprüche 1 bis 12, wobei
- die Versteifungsstücke aus einer Nickel-Titan-Legierung oder einem Edelstahlmaterial gebildet sind und/oder
- der Multilumendraht aus einem thermoplastischen Kunststoffmaterial gebildet ist und/oder
- die Abstandsstücke aus einem thermoplastischen Kunststoffmaterial oder einem duroplastischen Kunststoffmaterial oder einem Keramikmaterial gebildet sind und/oder
- Magnetresonanz-Markerpartikel in den Führungsdraht eingebracht sind.

14. Führungsdraht nach einem der Ansprüche 1 bis 13, wobei wenigstens eines der Versteifungsstücke an einem oder beiden Enden verrundet und/oder in seiner Biegesteifigkeit verringert ist.

## Claims

1. Guidewire configured for use in medical magnetic resonance applications, comprising
- a multi-lumen wire (1) composed of an electrically nonconductive plastic material, said wire extending continuously from a proximal end region to a distal end region of the guide wire and having at least two separate, axially extending hollow channels (2₁, ..., 2ₙ), wherein an axially alternating sequence of rod-shaped, elastic stiffening pieces (3) composed of an electrically conductive, nonmagnetic material having higher bending stiffness than the plastic material of the multi-lumen wire and electrically nonconductive spacer pieces (4) is arranged at least in one of the hollow channels of the multi-lumen wire, and/or
- coaxial wires (7₁, ..., 7ₙ) arranged coaxially one inside another and extending continuously from a proximal end region to a distal end region of the guide wire, wherein at least two of the coaxial wires (7₂, 7₃) have a hollow tube shape and are constructed from an axially alternating sequence of rod-shaped, elastic stiffening pieces (3) composed of an electrically conductive, nonmagnetic material and electrically nonconductive spacer pieces (4).

2. Guidewire as claimed in claim 1, wherein
- each stiffening piece has a greater length than each spacer piece and/or
- the length of each stiffening piece is in the range of between 1 cm and 15 cm and/or
- the length of each spacer piece is in the range of between 0.1 mm and 5 cm.

3. Guidewire as claimed in claim 1 or 2, wherein the stiffening pieces and the spacer pieces are arranged loosely successively.

4. Guidewire as claimed in any of claims 1 to 3, wherein a continuous tension rod (6) composed of an electrically nonconductive plastic material is arranged at least in one of the hollow channels.

5. Guidewire as claimed in claim 4, wherein the hollow channels include a central hollow channel, in which the continuous tension rod composed of electrically nonconductive plastic material is arranged.

6. Guidewire as claimed in any of claims 1 to 5, wherein at least one of the coaxial wires is formed by a continuous tension rod (6) composed of an electrically nonconductive plastic material.

7. Guidewire as claimed in claim 6, wherein the coaxial wires include a central coaxial wire, which is formed by the continuous tension rod composed of an electrically nonconductive plastic material.

8. Guidewire as claimed in any of claims 1 to 7, wherein
- the hollow channels include a central hollow channel, in which the alternating sequence of the stiffening pieces and spacer pieces is arranged, or
- the coaxial wires include a central coaxial wire, which is formed by the alternating sequence of the stiffening pieces and spacer pieces.

9. Guidewire as claimed in any of claims 1 to 8, wherein for a plurality of the hollow channels and/or coaxial wires the alternating sequence of the stiffening pieces and spacer pieces is arranged and the stiffening pieces and spacer pieces in a first of said hollow channels and/or coaxial wires are arranged axially offset relative to the stiffening pieces and spacer pieces in a second of said hollow channels and/or coaxial wires.

10. Guidewire as claimed in claim 9, wherein the spacer pieces are arranged in a manner axially free of overlap in the guide wire.

11. Guidewire as claimed in any of claims 1 to 10, wherein the hollow channels include a plurality of eccentric hollow channels, in which the alternating sequence of the stiffening pieces and spacer pieces is arranged.

12. Guidewire as claimed in any of claims 1 to 11, wherein the multi-lumen wire or an outermost one of the coaxial wires is surrounded by a shrink-on sleeve (5) or is provided with a hydrophilic coating or an antithrombogenic coating on the exterior.

13. Guidewire as claimed in any of claims 1 to 12, wherein
- the stiffening pieces are formed from a nickel-titanium alloy or a high-grade steel material and/or
- the multi-lumen wire is formed from a thermoplastic material and/or
- the spacer pieces are formed from a thermoplastic material or a thermosetting plastic material or a ceramic material and/or
- magnetic resonance marker particles are introduced into the guide wire.

14. Guidewire as claimed in any of claims 1 to 13, wherein at least one of the stiffening pieces is rounded and/or reduced in its bending stiffness at one or both ends.

## Revendications

1. Fil-guide configuré pour être utilisé dans des applications médicales de résonance magnétique, avec
- un fil multi-puissance (1) constitué d'un matériau plastique non conducteur d'électricité, qui s'étend en continu d'une extrémité proximale à une extrémité distale du fil-guide et présente au moins deux canaux creux séparés, s'étendant axialement (2₁,..., 2ₙ), sachant qu'est disposée, au moins dans un des canaux creux du fil multi-puissance, une suite alternant axialement de morceaux raidisseurs (3) élastiques, en forme de tiges, constitués d'un matériau non magnétique, conducteur d'électricité avec une rigidité à la flexion supérieure à celle du matériau plastique du fil multi-puissance et des morceaux espaceurs (4) non conducteurs d'électricité, et/ou
- des fils coaxiaux (7₁,..., 7ₙ) logés coaxialement les uns dans les autres, s'étendant d'une extrémité proximale à une extrémité distale du fil-guide, sachant qu'au moins deux des fils coaxiaux (7₂, 7₃) possèdent une forme de tube creux et sont formés à partir d'une suite alternant axialement de morceaux raidisseurs (3) élastiques, en forme de tubes, constitués d'un matériau non magnétique, conducteur d'électricité et de morceaux espaceurs (4) non conducteurs d'électricité.

2. Fil-guide selon la revendication 1, sachant que
- chaque morceau raidisseur possède une longueur supérieure à celle de chaque morceau espaceur et/ou
- la longueur de chaque morceau raidisseur est comprise entre 1 cm et 15 cm et/ou
- la longueur de chaque morceau espaceur est comprise entre 0,1 mm et 5 cm.

3. Fil-guide selon la revendication 1 ou 2, sachant que les morceaux raidisseurs et les morceaux espaceurs sont disposés consécutivement de façon lâche.

4. Fil-guide selon l'une des revendications 1 à 3, sachant qu'une tige de traction (6) continue composée d'un matériau plastique non conducteur d'électricité est disposée dans au moins un des canaux creux.

5. Fil-guide selon la revendication 4, sachant que fait partie des canaux creux un canal creux central dans lequel est disposée la tige de traction continue, composée d'un matériau plastique non conducteur d'électricité.

6. Fil-guide selon l'une des revendications 1 à 5, sachant qu'au moins un des fils coaxiaux est formé d'une tige de traction (6) continue, constituée d'un matériau plastique non conducteur d'électricité.

7. Fil-guide selon la revendication 6, sachant que fait partie des fils coaxiaux un fil coaxial central qui est formé par la tige de traction continue, composée d'un matériau plastique non conducteur d'électricité.

8. Fil-guide selon l'une des revendications 1 à 7, sachant que
- fait partie des canaux creux un canal creux central dans lequel est disposée la suite alternée des morceaux raidisseurs et morceaux espaceurs, ou
- fait partie des fils coaxiaux un fil coaxial central qui est formé par la suite alternée des morceaux raidisseurs et des morceaux espaceurs.

9. Fil-guide selon l'une des revendications 1 à 8, sachant que la suite alternée des morceaux raidisseurs et des morceaux espaceurs est disposée pour plusieurs des canaux creux et/ou fils coaxiaux et que les morceaux raidisseurs et les morceaux espaceurs dans un premier de ces canaux creux et/ou fils coaxiaux sont disposés décalés axialement par rapport aux morceaux raidisseurs et morceaux espaceurs dans un deuxième de ces canaux creux et/ou fils coaxiaux.

10. Fil-guide selon la revendication 9, sachant que les morceaux espaceurs dans le fil-guide sont disposés axialement sans chevauchement.

11. Fil-guide selon l'une des revendications 1 à 10, sachant que fait partie des canaux creux une pluralité de canaux creux décentrés dans lesquels est disposée la suite alternée des morceaux raidisseurs et des morceaux espaceurs.

12. Fil-guide selon l'une des revendications 1 à 11, sachant que le fil multi-puissance ou un des fils coaxiaux les plus extérieurs est entouré d'une gaine thermorétractable (5) ou est pourvu sur le côté extérieur d'un revêtement hydrophile ou d'un revêtement antithrombogène.

13. Fil-guide selon l'une des revendications 1 à 12, sachant que
- les morceaux raidisseurs sont formés d'un alliage nickel-titane ou d'un acier inoxydable et/ou
- le fil multi-puissance est formé d'un matériau thermoplastique et/ou
- les morceaux espaceurs sont formés d'un matériau thermoplastique ou d'un matériau duroplastique ou d'un matériau céramique et/ou
- des particules marqueurs de résonance magnétique sont incorporées au fil-guide.

14. Fil-guide selon l'une des revendications 1 à 13, sachant qu'au moins un des morceaux raidisseurs est arrondi et/ou présente une rigidité à la flexion diminuée à l'une des extrémités ou aux deux.
